# EUROPEAN PATENT SPECIFICATION

(11) **EP 0 661 047 B1**
(45) Date of publication and mention of the grant of the patent: **28.01.1998**
(21) Application number: 94201878.9
(22) Date of filing: 30.06.1994
(51) Int. Cl.: A61K 31/135, A61K 47/00

(54) **Pharmaceutical formulation containing diphenhydramine, talc, calamine and oat extract in an emulsified vehicle and uses thereof**
Pharmazeutische Zusammensetzung enthaltend Diphenhydramin, Talc, Zinkoxid und Haferextrakt in Emulsionsträger, sowie deren Verwendung
Composition pharmaceutique contenant la diphenhydramine, du talc, de l'oxyde de zinc et de l'extrait d'avoine dans un véhicle emulsionné, et leurs utilisations

(30) Priority: 31.12.1993 ES 9302734
(43) Date of publication of application: 05.07.1995
(73) Proprietor: LABORATORIOS CUSI, S.A., 08320 El Masnou (Barcelona) (ES)
(72) Inventor: Vallet Mas, Alberto, E-08022 Barcelona (ES); Coll Colomer, Jordi, E-08012 Barcelona (ES)
(74) Representative: Ungria Lopez, Javier

(56) References cited:
- US-A- 4 014 995
- US-A- 4 018 918
- US-A- 4 929 619
- FED.REGIST., vol.55, no.119, 1990 pages 25204 - 25232 JAMES S. BENSON 'skin protectant drug products for over-the -counter human use'

## Description

The present invention refers to an antiallergic, hydrophilous, dermatological topical pharmaceutical preparation useful to treat dermatitis, exanthemas, insect bites, rashes, eczemas, urticaria and minor skin irritations accompanied by itching and/or pain.

Specifically, the preparation contains diphenhydramine hydrochloride in an emulsified vehicle that includes oat extract, talc and at least one of calamine or zinc oxide, for topical dermatological use.

The topical antihistamines used to treat allergic-type dermatosis accompanied by itching and/or pain are included alone or combined with other active components, generally in the form of a solution, fluid or semisolid emulsion, gel or powder diluted in talc.

In the case of solutions, emulsions or gels, the topical antihistamines are never combined with oat flour (colloidal oat extract) or hydrated magnesium silicate (talc), ingredients which appear in the present invention and that contribute to alleviate itching, aside from reducing inflammation and facilitate gentle spreading of the preparation over damaged areas, aspects to be well kept in mind in skin affections that are accompanied by itching and/or pain.

Antihistamines diluted in talc to be sprinkled over the lesion are not associated either with an oat derivative. Besides, in this type of product there are risks of irregular distribution and of producing a cloud of powder that can cause respiratory disorders after inhalation thereof.

US-A-4929619 discloses a method of providing local and anti-infective treatment to a human or animal tissue area by application to the affected tissue area of an effective amount of a composition containing 0.5-5% by weight of selected antihistaminic agents in pharmaceutically acceptable topical vehicles including liquids, lotions, aerosols, creams, gels and ointments.

Fed. Regist. vol. 55, no 119, pages 25204-25232 describes the properties of several components of dermatological compositions, as colloidal oat extract, zinc oxide, and talc. In respect of colloidal oat, this document discloses that it a generally known secure and efficaceous product in the treatment of dermatological affections and that colloidal oatmeal is a skin protectant in preparations for dermatological use. Regarding talc, it is stated that it is known to have a skin-protecting activity due to its "barrier" properties, its insolubility in water and emollience. It is described as a typical product for treating skin irritations as for example diaper rash. Further, zinc oxide is identified as a known skin protectant and the most convenient percentages thereof in various formulations are given. The same as talc, zinc oxyde is also defined as especially suitable for treating and/or preventing diaper rash.

Despite the well known advantageous therapeutic and/or preventive properties of, on the one hand, diphenhydramine and, on the other, colloidal oat extract, talc and zinc oxide, no dermatological formulation containing all said ingredients has been described so far.

A problem common to conventional compositions containing talc, is the irregular distribution i.e. the lack of homgeneity of the composition.

The present invention is intended to overcome the aforementioned drawbacks of conventional compositions, by a new composition as claimed. The invention refers to an antiallergic-antipruritic topical pharmaceutical preparation that contains an antihistamine combined with oat flour (colloidal oat extract) in an emulsified vehicle that also includes calamine and talc or other drying, softening, emollient or protective ingredients. The vehicle is an emulsion of a fatty phase in an aqueous phase.

Oat flour (colloidal oat extract) is a substance with absorption capacity and that provides the skin with emollience.

Including calamine in the composition allows the product to be given its astringent and decongestant power.

To reinforce the properties of the active principle it is very useful to add softeners, protective agents, astringents and decongestants. Thus, it is particularly interesting to add talc, a hydrated magnesium silicate, well known and amply used as a softener and protective agent, in different dermatosis, due to its adherence and soft touch.

Talc is normally used in powder form in suitable containers to sprinkle the same, but this form of application has some inconveniences, such as for example irregular distribution and the formation of a cloud of powder that is easy to inhale and that can cause problems in the respiratory tract, particularly in children.

Upon being included in the emulsified vehicle of the present invention, talc is easily distributed over the surface to be treated without producing dust.

Including an antihistamine such as diphenhydramine hydrochloride, amply used due to its antiallergic and antipruritic properties, to active bases such as those indicated reinforces the soothing power of said active principle due to the coolness given to the aqueous emulsified vehicle and due to the softening, emollient and astringent action of the combination of the rest of the cited components.

The compositions of the present invention also include other ingredients normally used in dermatological topical preparations, such as for example, viscosity modifying agents, emulsifying agents, consistency regulators, protective agents, preservatives, emollients, antioxidants and chelating agents.

The compositions of the present invention can include substances used in microencapsulation, such as for example biocompatible polymers and different lipidic substances.

Including any representative of the above mentioned groups of compounds depends on the characteristics to be given to the final preparations. Choosing one compound or another depends on the physical, physicochemical and chemical characteristics of the rest of the components of the formulation in order to obtain a stable, well tolerated and therapeutically effective preparation.

Carboxyvinyl polymers, cellulose derivatives and gums, among others, can be cited as viscosity modifying agents. These compounds are normally used at some levels between 0.01 and 0.5%.

Keto-stearyl alcohol combined with non-ionic or anionic emulsifying agents, polyethyleneglycol stearate, polyglycol esters and polyoxyethylenesorbitan stearates, oleates or laurates can be cited among emulsifying agents. These compounds are normally used at some levels between 0.1 and 10%.

Cetyl alcohol, stearyl alcohol, keto-stearyl alcohol and stearic acid, among others, can be included as consistency regulators. The most normal levels of these compounds vary between 0.25 and 5%.

Dimethicones and cyclomethicones, among others, can be included as protective ingredients. These compounds are normally used at some levels between 0.1 and 10%.

Parabens, solutions of parabens in phenoxyethanol (Phenonip®), chloroisothiazolinone and methylchloroisothiazolinone solution (Kathon CG®), imidazolidinylurea (Germall 115®), among others, can be cited as preservatives. These compounds are typically used at some levels between 0.01 and 0.6% (Kathon CG® as a maximum of 0.15%.)

Lanolin and derivatives, glycerol and petrolate, among others, can be cited as emollients. These compounds are typically used at some levels between 0.05 and 10%.

BHT, BHA, tocopherol or esters thereof and sodium sulfite, among others can be included as antioxidants. These compounds are normally used at some levels between 0.01 and 2%.

As chelating agents, we can cite citric acid and disodium salt of ethylenediaminetetraacetic acid, EDTA. These compounds are included to improve the action of the preservatives. These compounds are typically used at some levels between 0.01 and 2%.

A polyacrylic, polylactic, polyglycol derivative, a polylactic-glycol copolymer, a polyanhydride, a polyamide, a poly(alpha-amino acid), cellulose polymers, natural polymers, or a mixture of two or more of the cited compounds can be included as biocompatible polymers. These compounds are typically used at some levels between 0.1 and 5.0%.

Coconut oil, ethoxylated oleic glycerides, diethyleneglycol monoethyl ether, C₈-C₁₀ ethoxylated glycerides, phospholipids, natural oils or petroleum derivatives or a mixture of several of them can be used as lipidic substances. These compounds are normally used at some levels between 0.01 and 20%.

In accordance with the present invention, the resulting formulation includes between 0.25 and 2% diphenhydramine hydrochloride and between 0.1 and 10% of a colloidal oat extract in en emulsified vehicle that also includes, between 0.05 and 10% calamine or zinc oxide and between 1 and 10% talc or other drying, softening, emollient or protective ingredients.

The amount of preparation that can be administered to the patient depends on his age as well as his general state of health and on the severity and type of disease suffered from. Though the doctor is to establish the dosage, it is recommended that the application of the formulations included in the present invention be done 2 to 4 times a day, spreading the preparation as a fine layer.

The formulations included in the present invention can be contained in the containers normally used for this type of preparation, depending on the type of resulting pharmaceutical form.

### EMBODIMENTS OF THE INVENTION

The following formulations are given as representative examples of the compositions included in the present invention and they must not be considered as limitations of the scope of the same.

| EXAMPLE Nº 1. Fluid emulsion | |
|---|---|
| Substance | Amounts per 100 g |
| Carbomer® 940 | 0.17 g. |
| Phenonip | 0.60 g. |
| Colloidal oat extract | 5.00 g. |
| Talc | 10.00 g. |
| Keto-stearyl alcohol | 0.60 g. |
| Cyclomethicone | 0.50 g. |
| Dimethicone | 0.50 g. |
| Polyoxyethylenated cetyl emulsifying agent | 2.50 g. |
| Triethanolamine | 0.20 g. |
| Ethoxylated lanolin | 0.40 g. |
| Kathon® CG | 0.10 g. |
| Calamine | 2.00 g. |
| Diphenhydramine hydrochloride | 1.00 g. |
| Purified water q.s. | 100.00 g. |

### Preparation method

### Aqueous phase

Place in a suitable container the purified water, except the amount needed to dissolve the diphenhydramine hydrochloride, add Carbomer 940® and Phenonip® (50% of the total amount) and stir until swelling of the Carbomer® 940. Then add, with stirring, the colloidal oat extract and the talc and heat to 70º C.

### Fatty phase

In a separate container, melt and stir until homonegeity keto-stearyl alcohol, cyclomethicone, dimethicone, emulsifying agent and the rest of the Phenonip. Heat to 70º C.

### Preparation of the emulsion

The two phases at 70º C, add the fatty phase to the aqueous phase with stirring and once the mixture is homogeneous, add the triethanolamine and the ethoxylated lanolin, maintaining the stirring.

Refrigerate and add, at 40º C, the Kathon CG and then the calamine. Finally, once the suspension is homogenous and the temperature is about 30º C add the diphenhydramine hydrochloride solution in twice its weight of water.

| EXAMPLE Nº 2. Fluid emulsion | |
|---|---|
| Substance | Amounts per 100 g. |
| Palmito-polyglycol stearate | 7.00 g. |
| Stearic acid | 1.00 g. |
| Mono,di palmito-glyceryl stearate | 1.00 g. |
| 2-octyl dodecyl myristate | 5.00 g. |
| Borage oil | 0.50 g. |
| Avocado oil | 3.00 g. |
| Alpha-bisabolol | 0.10 g. |
| Tocopherol acetate | 1.00 g. |
| Panthenol | 1.00 g. |
| Carbomer® 941 | 0.10 g. |
| Triethanolamine | 0.20 g. |
| Kathon® CG | 0.10 g. |
| Antioxidant | 0.10 g. |
| Colloidal oat extract | 5.00 g. |
| Talc | 10.00 g. |
| Calamine | 2.00 g. |
| Diphenhydramine hydrochloride | 1.00 g. |
| Perfume | 0.20 g. |
| Purified water q.s. | 100.00 g. |

### Preparation method

### Aqueous phase

In a suitable container, swell in purified water, except the amount needed to dissolve the diphenhydramine hydrochloride, Carbomer® 941, add the panthenol and homogenize. Add the colloidal oat extract and the talc. Heat to 70º C.

### Fatty phase

In a separate container, melt and homogenize palmito-polyglycol stearate, stearic acid, mono, di palmito-glycerol stearate, 2-octyl dodecyl myristate, borage oil, avocado oil, alpha-bisabolol, tocopherol acetate and antioxidant. Heat to 70º C.

### Preparation of the emulsion

The two phases at 70º C, add the fatty phase to the aqueous phase with stirring and once the mixture is homogeneous, add the triethanolamine. Keep stirring. Refrigerate and add, at 40º C, Kathon CG and then the calamine. Finally, once the suspension is homogeneous and the temperature is about 30º C, add the diphenhydramine hydrochloride solution in twice its weight of water and the perfume.

| EXAMPLE Nº 3. Semisolid emulsion | |
|---|---|
| Substance | Amounts per 100 g. |
| Palmito-polyglycol stearate | 12.00 g. |
| Stearic acid | 1.00 g. |
| Moni, di palmito-glyceryl stearate | 2.00 g. |
| 2-octyl dodecyl myristate | 10.00 g. |
| Borage oil | 0.50 g. |
| Alpha-bisabolol | 0.10 g. |
| Tocopherol acetate | 3.00 g. |
| Panthenol | 1.00 g. |
| Kathon® CG | 0.10 g. |
| Colloidal oat extract | 5.00 g. |
| Talc | 10.00 g. |
| Calamine | 2.00 g. |
| Diphenhydramine hydrochloride | 1.00 g. |
| Perfume | 0.20 g. |
| Purified water q.s. | 100.00 g. |

### Preparation method

Place in a suitable container the purified water, except the amount needed to dissolve the diphenhydramine hydrochloride, add the panthenol and homogenize. Add the colloidal oat extract and the talc. Heat to 70º C.

### Fatty phase

In a separate container, melt and homogenize the palmito-polyglycol stearate, stearic acid, mono, di-palmito-glyceryl stearate, 2-octyl dodecyl myristate, borage oil, alpha-bisabolol and tocopherol acetate. Heat to 70º C.

### Preparation of the emulsion

The two phases at 70º C, add the fatty phase to the aqueous phase with stirring and keep stirring.

Refrigerate and add, at 40º C, the Kathon® CG and then the calamine. Finally, once the suspension is homogenous and the temperature is about 30º C, add the diphenhydramine hydrochloride solution in twice its weight in water and the perfume.

| EXAMPLE Nº 4. Fluid emulsion | |
|---|---|
| Substance | Amounts per 100 g. |
| Carbomer® 940 | 0.17 g. |
| Phenonip | 0.60 g. |
| Colloidal oat extract | 5.00 g. |
| Talc | 10.00 g. |
| Keto-stearyl alcohol | 0.60 g. |
| Cyclomethicone | 0.50 g. |
| Dimethicone | 0.50 g. |
| Polyoxyethylenated cetyl emulsifying agent | 2.50 g. |
| Triethanolamine | 0.20 g. |
| Ethoxylated lanolin | 0.40 g. |
| Kathon® CG | 0.10 g. |
| Calamine | 2.00 g. |
| Colloidal suspension 2% of nanocapsules or nanospheres of diphenhydramine | 25.00 ml. |
| Purified water q.s. | 100.00 g. |

### Preparation method

### Aqueous phase

Place in a suitable container the purified water, add the Carbomer 940 and the Phenonip (50 % of the total amount) and stir until swelling of the Carbomer 940. Then add, with stirring, the colloidal oat extract and the talc and heat to 70º C.

### Fatty phase

In a separate container, melt and stir to homogeneity keto-stearyl alcohol, cyclomethicone, dimethicone, emulsifying agent and the rest of the Phenonip. Heat to 70º C.

### Preparation of the emulsion

The two phases at 70º C, add the fatty phase to the aqueous phase with stirring and once the mixture is homogeneous, add the triethanolamine and the ethoxylated lanolin. Keep stirring.

Refrigerate and add, at 40º C, the Kathon® CG and then the calamine. Finally, once the suspension is homogeneous and the temperature is about 30º C, add the colloidal suspension 2% of nanocapsules or nanospheres of diphenhydramine.

## Claims

1. Pharmaceutical formulation containing diphenhydramine, talc, and at least one of zinc oxide or calamine and oat extract, in an emulsified vehicle, for dermatological use, characterized in that the vehicle is an emulsion of a fatty phase in an aqueous phase and in that it comprises:
- 0.25-2.0% diphenhydramine hydrochloride or base,
- 0.1-10.0% colloidal oat extract,
- 0.5-10% calamine or zinc oxide,
- 1-20% talc
- 0.1-10% emulsifying agent,
- optionally, 0.01-0.5% viscosity modifying agent,
- optionally, 0.25-5.0% consistency regulator,
- optionally, 0.1-10% water-repellent protector,
- optionally, 0.01-0.6% preservative,
- optionally, 0.05-10.0% emollient,
- optionally, 0.01-2.0% antioxidant,
- optionally, 0.01-2.0% chelating agent,
- optionally, 0.01-20% lipidic substances for microencapsulation of the diphenhydramine in the formulation,
- optionally, 0.1-5.0% biocompatible polymers for microencapsulation of the diphenhydramine in the formulation.

2. A formulation, according to claim 1, characterized in that the emulsifying agent is selected from the group comprised of polyoxyethylenated cetyl alcohol, sodium cetyl-sulfate, sodium cetyl-stearyl sulfate, palmito-glycol stearate, polyethyleneglycol stearate, polyglycol ethers and polyoxyethylenated sorbitan esters.

3. A formulation, according to claim 1, characterized in that the viscosity modifying agent is selected from the group comprised of carboxyvinyl polymer, metylcellulose, carboxymetnylcellulose, hydroxyethylcellulose, hydroxypropylmethylcellulose and gum.

4. A formulation, according to claim 1, characterized in that the consistency regulator is selected from the group comprised of cetyl alcohol, stearyl alcohol, keto-stearyl alcohol and stearic acid, as well as by any of the emulsifying agents cited in claim 2.

5. A formulation according to claim 1, characterized in that the protector is selected from the group comprised of dimethicones and cyclomethicones.

6. A formulation according to claim 1, characterized in that the preservative is selected from the group comprise of paraban, solutions of paraben in phenoxyethanol, chloroisothiazolinone and methylchloroisothiazolinone solution and imidazolidinylurea.

7. A formulation according to claim 1, characterized in that the emollient is selected from the group comprised of lanolin and derivatives, glycerol and petrolate.

8. A formulation according to claim 1, characterized in that the antioxidant is selected from the grupp comprised of BHT, BHA, tocopherol (or esters thereof) and sodium sulfite.

9. A formulation according to claim 1, characterized in that the chelating agent is selected from the group comprised of citric acid and disodium salt of ethylenediamine tetraacetic acid (EDTA.)

10. A formulation according to claim 1, characterized in that the excipient is selected from among agents that allow the diphenyhydramine of the formulation to be microencapsulated, such as the lipidic substances (coconot oil, ethoxylated oleic glyceride, diethyeleneglycol monoethyl ether, C₈-C₁₀ ethoxylated glyceride, phospholipids, natural oils or petroleum derivatives or a mixture of several of them) and biocompatible polymers (polyacrylic, polylactic, polyglycol derivative, a polylactic-glycol copolymer, a polyanhydride, a polyamide, a poly-alpha-amino acid, cellulose polymers, natural polymers or a mixture of two or more of the cited compounds.

11. Use of the formulations of above claims 1 to 10 for the manufacture of a medicament for topical treatment of allergic-type dermatosis accompanied by itching and/or pain.

## Patentansprüche

1. Pharmazeutische Zubereitung enthaltend Diphenhydramin, Talk, und mindestens einen Bestandteil ausgewählt aus Zinkoxid und Calamin, und Haferextrakt, in einem emulgierten Träger, zur dermatologischen Verwendung, **dadurch gekennzeichnet,** daß der Träger eine Emulsion von einer fettartigen Phase in einer wäßrigen Phase ist, und dadurch, daß sie umfaßt:
0,25-2,0 % Diphenhydramin-Hydrochlorid oder -Base,
0,1-10,0 % kolloidaler Haferextrakt,
0,5-10 % Calamin oder Zinkoxid,
1-20 % Talk,
0,1-10 % Emulgator,
gegebenenfalls 0,01-0,5 % Mittel zur Veränderung der Viskosität,
gegebenenfalls 0,25-5,0 % Konsistenzregler,
gegebenenfalls 0,1-10 % wasserabweisendes Schutzmittel,
gegebenenfalls 0,01-0,6 % Konservierungsmittel,
gegebenenfalls 0,05-10,0 % Erweichungsmittel,
gegebenenfalls 0,01-2,0 % Antioxidationsmittel,
gegebenenfalls 0,01-2,0 % Chelatbildner,
gegebenenfalls 0,01-20 % Lipidsubstanzen zur Mikroverkapselung des Diphenhydramins in der Zubereitung,
gegebenenfalls 0,1-5,0 % bioverträgliche Polymere zur Mikroverkapselung des Diphenhydramins in der Zubereitung.

2. Zubereitung nach Anspruch 1, **dadurch gekennzeichnet,** daß der Emulgator ausgewählt ist aus der Gruppe, die sich aus polyoxyethyleniertem Cetylalkohol, Natriumcetylsulfat, Natriumcetyl-stearylsulfat, Palmitoglycolstearat, Polyethylenglycolstearat, Polyglycolethern und polyoxyethylenierten Sorbitanestern zusammensetzt.

3. Zubereitung nach Anspruch 1, **dadurch gekennzeichnet,** daß das Mittel zur Veränderung der Viskosität ausgewählt ist aus der Gruppe, die sich aus Carboxyvinylpolymer, Methylcellulose, Carboxymethylcellulose, Hydroxyethylcellulose, Hydroxypropylmethylcellulose und Gummi zusammensetzt.

4. Zubereitung nach Anspruch 1, **dadurch gekennzeichnet,** daß der Konsistenzregler ausgewählt ist aus der Gruppe, die sich zusammensetzt aus Cetylalkohol, Stearylalkohol, Ketostearylalkohol und Stearinsäure, sowie durch jeden der in Anspruch 2 aufgeführten Emulgatoren.

5. Zubereitung nach Anspruch 1, **dadurch gekennzeichnet,** daß das Schutzmittel ausgewählt ist aus der Gruppe, die sich aus Dimethiconen und Cyclomethiconen zusammensetzt.

6. Zubereitung nach Anspruch 1, **dadurch gekennzeichnet,** daß das Konservierungsmittel ausgewählt ist aus der Gruppe, die sich aus Paraben, Lösungen von Paraben in Phenoxyethanol, Chlorisothiazolinon und Methylchlorisothiazolinonlösung und Imidazolidinylharnstoff zusammensetzt.

7. Zubereitung nach Anspruch 1, **dadurch gekennzeichnet,** daß das Erweichungsmittel ausgewählt ist aus der Gruppe, die sich aus Lanolin und Derivaten davon, Glycerin und Petrolat zusammensetzt.

8. Zubereitung nach Anspruch 1, **dadurch gekennzeichnet,** daß das Antioxidationsmittel ausgewählt ist aus der Gruppe, die sich aus BHT, BHA, Tocopherol (oder Estern davon) und Natriumsulfit zusammensetzt.

9. Zubereitung nach Anspruch 1, **dadurch gekennzeichnet,** daß der Chelatbildner ausgewählt ist aus der Gruppe, die sich aus Citronensäure und dem Dinatriumsalz von Ethylendiamintetraessigsäure (EDTA) zusammensetzt.

10. Zubereitung nach Anspruch 1, **dadurch gekennzeichnet,** daß der Träger ausgewählt ist aus Mitteln, welche gestatten, daß das Diphenhydramin der Zubereitung mikroverkapselt wird, wie etwa die Lipidsubstanzen (Kokosnußöl, ethoxyliertes Ölsäureglycerid, Diethylenglycolmonoethylether, C₈-C₁₀-ethoxyliertes Glycerid, Phospholipide, natürliche Öle oder Petroleumderivate oder ein Gemisch aus mehreren von diesen) und bioverträgliche Polymere (Polyacryl-, Polymilchsäure-, Polyglycol-Derivat, ein Polymilchsäure-Glycol-Copolymer, ein Polyanhydrid, ein Polyamid, eine Poly-alpha-aminosäure, Cellulosepolymere, natürliche Polymere oder ein Gemisch aus zwei oder mehreren der genannten Verbindungen).

11. Verwendung der Zubereitungen der vorstehenden Ansprüche 1-10 zur Herstellung eines Arzneimittels zur topischen Behandlung einer allergieartigen Dermatose, die mit Jucken und/oder Schmerzen verbunden ist.

## Revendications

1. Formulation pharmaceutique contenant de la diphénhydramine, du talc et au moins de l'oxyde de zinc ou de la calamine et de l'extrait d'avoine dans un véhicule émulsifié, à usage dermatologique, caractérisée en ce que le véhicule est une émulsion d'une phase graisseuse dans une phase aqueuse et en ce qu'elle comprend :
- 0,25 à 2,0 % de chlorhydrate de diphénhydramine ou de diphénhydramine base
- 0,1 à 10,0 % d'extrait d'avoine colloïdal,
- 0,5 à 10 % de calamine ou d'oxyde de zinc,
- 1 à 20 % de talc,
- 0,1 à 10 % d'agent émulsifiant,
- facultativement, 0,01 à 0,5 % d'agent modificateur de la viscosité,
- facultativement, 0,25 à 5,0 % de régulateur de la consistance,
- facultativement, 0,1 à 10 % d'agent protecteur hydrofuge,
- facultativement, 0,01 à 0,6 % d'agent de conservation,
- facultativement, 0,05 à 10,0 % d'émollient,
- facultativement, 0,01 à 2,0 % d'antioxydant,
- facultativement, 0,01 à 2,0 % d'agent chélatant,
- facultativement, 0,01 à 20 % de substances lipidiques pour la microencapsulation de la diphénhydramine dans la formulation,
- facultativement, 0,1 à 5,0 % de polymères biocompatibles pour la microencapsulation de la diphénhydramine dans la formulation.

2. Formulation selon la revendication 1, caractérisée en ce que l'agent émulsifiant est sélectionné dans le groupe comprenant l'alcool cétylique polyoxyéthyléné, le cétylsulfate de sodium, le cétylstéarylsulfate de sodium, le stéarate de palmitoglycol, le stéarate de polyéthylèneglycol, les éthers de polyglycol et les éthers de sorbitanne polyoxyéthylénés.

3. Formulation selon la revendication 1, caractérisée en ce que l'agent modificateur de la viscosité est sélectionné dans le groupe comprenant un polymère de carboxyvinyle, la méthylcellulose, la carboxyméthylcellulose, l'hydroxyéthylcellulose, l'hydroxypropylméthylcellulose et la gomme.

4. Formulation selon la revendication 1, caractérisée en ce que le régulateur de consistance est sélectionné dans le groupe comprenant l'alcool cétylique, l'alcool stéarylique, l'alcool cétostéarylique et l'acide stéarique, de même qu'un quelconque des émulsifiants cités à la revendication 2.

5. Formulation selon la revendication 1, caractérisée en ce que l'agent protecteur est sélectionné dans le groupe comprenant les diméthicones et les cyclométhicones.

6. Formulation selon la revendication 1, caractérisée en ce que l'agent de conservation est sélectionné dans le groupe comprenant para-hydroxybenzoate, solutions de para-hydroxybenzoate dans le phénoxyéthanol, solution de chloroisothiazolinone et de méthylchloroisothiazolinone et imidazolidinyl-urée.

7. Formulation selon la revendication 1, caractérisée en ce que l'émollient est sélectionné dans le groupe constitué par la lanoline et ses dérivés, le glycérol et la vaseline.

8. Formulation selon la revendication 1, caractérisée en ce que l'antioxydant est sélectionné dans le groupe comprenant BHT, BHA, le tocophérol (ou ses esters) et le sulfite de sodium.

9. Formulation selon la revendication 1, caractérisée en ce que l'agent chélatant est sélectionné dans le groupe comprenant l'acide citrique et le sel disodique de l'acide éthylènediaminotétracétique (EDTA).

10. Formulation selon la revendication 1, caractérisée en ce que l'excipient est sélectionné parmi des agents permettant la microencapsulation de la diphénhydramine de la formulation, tels que des substances lipidiques (huile de coco, glycéride oléique éthoxylé, éther monoéthylique de diéthylèneglycol, glycéride éthoxylé en C₈-C₁₀, phospholipides, huiles naturelles ou dérivés de pétrole ou mélange de plusieurs d'entre eux) et des polymères biocompatibles (dérivé polyacrylique, polylactique, polyglycolique, copolymère polylactiqueglycol, polyanhydride, polyamide, poly-α-aminoacide, polymères cellulosiques, polymères naturels ou mélange de deux ou plus des composés cités).

11. Utilisation des formulations selon les revendications 1 à 10 pour la préparation d'un médicament destiné au traitement topique des dermatoses de type allergique s'accompagnant de prurit et/ou de douleurs.
